# EUROPEAN PATENT SPECIFICATION

(11) **EP 2 638 148 B1**
(45) Date of publication and mention of the grant of the patent: **21.12.2016**
(21) Application number: 11754744.8
(22) Date of filing: 11.08.2011
(51) Int. Cl.: C12N 5/0793, C12N 5/09

(54) **GLIOMA STEM CELLS AND METHODS FOR OBTAINING THEM**
GLIOMSTAMMZELLEN UND VERFAHREN ZU IHRER GEWINNUNG
CELLULES SOUCHES DE GLIOME ET LEURS PROCÉDÉS D'OBTENTION

(30) Priority: 12.11.2010 EP 10290606
(43) Date of publication of application: 18.09.2013
(73) Proprietor: Commissariat à l'Énergie Atomique et aux Énergies Alternatives, 75015 Paris (FR); Centre National de la Recherche Scientifique, 75016 Paris (FR)
(72) Inventor: BOUSSIN, François, 92340 Bourg-la-Reine (FR); SILVESTRE, David, 75010 Paris (FR); CHNEIWEISS, Hervé, 75003 Paris (FR); JUNIER, Marie-Pierre, 75020 Paris (FR)
(74) Representative: Gevers & Orès
(86) International application number: PCT/IB2011/053585
(87) International publication number: WO 2012/063139

(56) References cited:
- HAKIN-SMITH V ET AL: "Alternative lengthening of telomeres and survival in patients with glioblastoma multiforme", THE LANCET, vol. 361, no. 9360, 8 March 2003 (2003-03-08), pages 836-838, XP004414613, LANCET LIMITED. LONDON, GB ISSN: 0140-6736, DOI: 10.1016/S0140-6736(03)12681-5
- SHERVINGTON ET AL: "Telomerase subunits expression variation between biopsy samples and cell lines derived from malignant glioma", BRAIN RESEARCH, vol. 1134, 23 February 2007 (2007-02-23), pages 45-52, XP022040125, ELSEVIER, AMSTERDAM, NL ISSN: 0006-8993, DOI: 10.1016/J.BRAINRES.2006.11.093
- LANGFORD L A ET AL: "Telomerase activity in human brain tumours", THE LANCET, vol. 346, no. 8985, 11 November 1995 (1995-11-11), pages 1267-1268, XP002587745, ISSN: 0140-6736
- GROBELNY JENNIFER V ET AL: "Effects of reconstitution of telomerase activity on telomere maintenance by the alternative lengthening of telomeres (ALT) pathway", HUMAN MOLECULAR GENETICS, vol. 10, no. 18, 1 September 2001 (2001-09-01), pages 1953-1961, XP002627978, ISSN: 0964-6906
- LEE J ET AL: "Tumor stem cells derived from glioblastomas cultured in bFGF and EGF more closely mirror the phenotype and genotype of primary tumors than do serum-cultured cell lines", CANCER CELL, vol. 9, no. 5, 1 May 2006 (2006-05-01), pages 391-403, XP002550739, CELL PRESS, US ISSN: 1535-6108, DOI: 10.1016/J.CCR.2006.03.030
- MAO XING-GANG ET AL: "Brain Tumor Stem-Like Cells Identified by Neural Stem Cell Marker CD15", TRANSLATIONAL ONCOLOGY, vol. 2, no. 4, December 2009 (2009-12), pages 247-257, XP002627979, ISSN: 1936-5233
- BEIER ET AL: "Identification of CD133-/telomerase low progenitor cells...", CELL MOL NEUROBIOL, 17 November 2010 (2010-11-17), XP002627980, Retrieved from the Internet: URL:http://www.springerlink.com/content/81 h523478421m2w2/ [retrieved on 2011-03-14]
- SILVESTRE D C ET AL: "Alternative lengthening of telomeres in human glioma stem cells", STEM CELLS 2011 ALPHAMED PRESS USA LNKD- DOI:10.1002/STEM.600, vol. 29, no. 3, March 2011 (2011-03), pages 440-451, XP002665134, ISSN: 1066-5099
- C ALTANER: 'Glioblastoma and stem cells Minireview' NEOPLASMA. vol. 55, no. 5, 01 January 2008, SK, pages 369 - 374, XP055121615 ISSN: 0028-2685

## Description

### Field of the invention

The present invention relates to a glioma stem cell population, methods for obtaining a glioma stem cell population and to the use of said glioma stem cell population.

### Background of the invention

Glioblastoma multiforme (GBM) is the most aggressive form of brain tumours with extremely poor prognosis (1). At present, radiotherapy remains a primary treatment for this malignancy. Nevertheless, even combined with surgery, the median survival of patients with GBM is less than 1 year (2), and this prognosis has not been significantly improved in the last 20 years (3).

Recently, stem-like cells have been evidenced in gliomas (Glioma Stem-like cells, GSCs) (4-5). These cells share characteristics with normal neural stem cells, such as the capacity for self-renewal and long-term proliferation, the formation of neurospheres and the ability to differentiate into multiple nervous system lineages. However, GSCs are different from normal neural stem cells in that the former express aberrantly differentiation markers, have abnormal karyotypes and are capable to form tumours when injected into immunodeficient mouse brain (5-6).

According to the current paradigm, GSCs are proposed to be highly radioresistant (7) and to survive to radiotherapy, repopulating the tumour afterwards (8).

It would therefore be highly advantageous to target these GSCs in order to prevent relapse of GBM in individuals.

Telomeres are specialized DNA-protein complexes found at the end of chromosomes and in humans they consist of linear tandem arrays of TTAGGG repeats ending in a single stranded G-rich 3' overhang that contributes to a higher-order terminal loop structure, the t loop. Together with their associated proteins, they play a critical role protecting chromosomes from end degradation and end-to-end fusions, and are essential for genomic integrity (9). A loss of telomeric DNA is found with each cell division in most somatic cells, mainly due to the end replication problem. Since short telomeres drive eukaryotic cells into replicative senescence, the maintenance of functional telomeres is crucial for continued proliferation. To circumvent this problem, cells maintain their telomeres usually by telomerase activity (10), which is a cellular reverse transcriptase that adds new telomere repeats onto the telomeres. Telomerase activity is found in germ cells, and in somatic stem and progenitor cells, but is lost during differentiation (11-12). Reactivation of telomere elongation activity is thought to be the most conspicuous feature of cancer cells, representing an obligate requirement for uncontrolled tumour growth. Nevertheless, it has been found that some cancer cells proliferate despite showing no discernible telomerase activity. Some of these cells employ a different telomere maintenance mechanism, termed alternative lengthening of telomeres (ALT) (13), which is a recombination mediated-telomerase independent-DNA replication process. About 10% of human cancers show the ALT phenotype, being abundant in sarcomas of mesenchymal origin and very rare in epithelial carcinomas (14). It has been reported that some glioma tumors are telomerase negative (15, 53, 54). In particular, a study has shown that the ALT phenotype is present in 25 % of glioblastoma multiforme tumors and associated with a better survival in patients. However, no difference in response to radiotherapy was found between ALT positive and ALT negative glioblastoma multiforme tumors (15).

It has been recently postulated that the stem cell potential of GSC would necessarily be associated with reactivation of telomerase as in normal neural stem cells, which has lead to the use of primary human glioblastoma tumor-initiating cells presenting a telomerase activity for testing the therapeutic efficiency of putative anti-glioblastoma drugs (18). However, complete eradication of glioblastoma multiforme using treatments deriving from such a screening has not been demonstrated yet. Indeed, it has not been shown that such GSCs were representative of the glioblastoma cells responsible for the frequent post-treatment glioblastoma multiforme relapse. Accordingly, alternative models are still needed.

### Summary of the invention

The present invention arises from the unexpected isolation and characterization, by the inventors, of adult human glioma stem cells (GSCs) maintaining their telomeres by an ALT pathway. This finding is particularly unexpected because it was considered in the prior art that telomerase expression in GSCs or in glioma cell lines was required for tumor formation (26).

Unexpectedly also, the inventors have found that the GSCs of the invention were more resistant to ionizing irradiation than GSCs presenting a telomerase activity. The GSCs of the invention are thus particularly advantageous in that they provide a model for radiation-resistant GSCs likely to be responsible for glioblastoma multiforme relapse.

Accordingly, the present invention relates to a glioma stem cell (GSC) population, wherein the glioma stem cells of the population do not present a telomerase activity.

The present invention also relates to a method for obtaining a glioma stem cell population, comprising:
- culturing cells dissociated from a glioma tumor sample obtained from an individual, in a neural stem cell culture medium comprising epidermal growth factor (EGF) and fibroblast growth factor 2 (FGF-2) to obtain neurosphere populations;
- determining that the tumor sample or the cells dissociated from the tumor sample do not present a telomerase activity and/or selecting a neurosphere population which do not present a telomerase activity;
thereby obtaining a glioma stem cell population.

The present invention also relates to a glioma stem cell population according to the invention which is obtainable by the method for obtaining a glioma stem cell population of the invention.

The present invention further relates to the use of a glioma stem cell population according to the invention for the generation of glioblastoma tumors in an animal. The present invention also relates to a method for generating gliobastoma tumors in an animal, comprising administering an effective quantity of a glioma stem cell population according to the invention to the animal.

The invention further relates to the use, in particular the *in vitro* or *ex vivo* use, of a glioma stem cell population according to the invention, for screening anti-glioblastoma or anti-ALT treatments. The present invention also relates to a method for screening anti-glioblastoma or anti-ALT treatments, comprising administering a glioblastoma or an ALT candidate treatment to an animal harboring glioblastoma tumors generated from the administration of a glioma stem cell population according to the invention and determining if the candidate treatment improves the condition of the animal.

The application discloses the use, in particular the *in vitro* or *ex vivo* use, of a glioma stem cell population according of the invention for studying ALT mechanism and pathogenesis, in particular for identifying potential biochemical targets of anti-ALT treatment or drugs, and for screening anti-ALT treatments.

### Detailed description of the invention

As intended herein the expression "glioma stem cells" or "GSCs" relates to cells presenting all, of: (i) the ability to self-renew in defined stem cell culture conditions, in particular neural stem cell culture conditions, (ii) the expression of normal neural stem cell markers, (iii) the ability to generate a differentiated progeny, and (iv) the capacity to generate brain tumors, in particular gliobastoma tumors, in animals. Glioma stem cells are notably described in references (4)-(5) and (26)-(28).

A "glioma stem cell population" according to the invention comprises glioma stem cells. Preferably, at least 50%, 60%, 70%, 80%, 90%, or 95%, and more preferably essentially all, of the cells of the glioma stem cell population of the invention are glioma stem cells.

Preferably, the glioma stem cells of the population according to the invention are human cells. The glioma stem cells of the population according to the invention are adult or non-fetal cells.

Preferably, stem cell culture conditions according to the invention do not comprise differentiation-inducing components, such as serum, in particular fetal bovine serum (FBS) or fetal calf serum (FCS).

Glioma stem cell populations according to the invention do not present a telomerase activity.

One of skill in the art is aware of numerous easily implementable methods for determining if a cell population presents a telomerase activity. By way of example, one of skill in the art may monitor telomerase enzymatic activity, telomerase RNA expression quantity, for instance by RT-PCR, or telomerase protein quantity, for instance by ELISA, as is notably described in the following Example.

Preferably, the glioma stem cells of the population according to the invention display heterogeneous telomere lengths and/or an intracellular colocalization of telomeric DNA and promyelocytic leukemia (PML) nuclear bodies.

Here again, one of skill in the art can easily determine whether the glioma stem cells of the population according to the invention display heterogeneous telomere lengths and/or an intracellular colocalization of telomeric DNA and promyelocytic leukemia (PML) nuclear bodies.

By way of example, heterogenous telomere lengths can be evidenced by performing a Southern blotting of telomere restriction fragments using a probe targeting telomeric sequences. Preferably, according to the invention, the length of telomeres as visualized by Southern blotting of telomere restriction fragments is said to be heterogenous if it is similar to that of SAOS-2 cells, a well-known ALT-osteosarcoma cell line notably described in reference (22).

PML nuclear bodies, in particular ALT-associated PML nuclear bodies are well known to one of skill in the art and are notably described in reference (25). Colocalization of telomeric DNA and promyelocytic leukemia (PML) nuclear bodies may for instance be determined by confocal microscopy.

Accordingly, the population of glioma stem cells of the invention preferably presents an alternative lengthening of telomeres (ALT). ALT is well known to one of skill in the art and is notably defined in reference (13).

Preferably, the glioma stem cell population according to the invention is liable to generate glioblastoma tumors upon administration to an animal.

An animal according to the invention is a non-human animal, more preferably a non-human mammal, such as a mouse, a rat, or a rabbit. Preferably also, the animal according to the invention is an immunocompromised or immunodeficient animal, *i.e.* an animal having an impaired or non-functional immune system, such as a non-obsese diabetic severe combined immunodeficient (NOD-SCID) mouse, in particular a NOD-SCID-IL2Rγ (NOG) mouse, such as defined in reference (50). Administration, according to the invention, of a population of glioma stem cells according to the invention to an animal according to the invention may notably be performed by an intracranial injection, in particular in the striatum region of the brain of the animal.

The generation of glioblastoma tumors according to the invention may notably be evidenced by Nestin marking of brain slices of the animal.

Preferably, the glioma stem cell population according to the invention is such that:
- less than 1% of the glioma stem cells of the population express CD133, and/or
- from 30% to 70% of the glioma stem cells of the population express CD15, and/or
- more than 90% of the glioma stem cells of the population express Sox2, and/or
- less than 20% of the glioma stem cells of the population express glial fibrillary acidic protein (GFAP), and/or
- less than 20% of the glioma stem cells of the population express microtubule-associated protein 2 (MAP2).

CD133, CD15, Sox2, GFAP, and MAP2 expression by glioma stem cells of the invention may be measured by numerous methods well-known to one of skill in the art, such as fluorescence-assisted cell sorting using fluorescently labeled antibodies.

Preferably, the glioma stem cell population according to the invention is the cell line deposited at the Collection Nationale de Culture de Micro-organismes (CNCM), Institut Pasteur, Paris, France, under the Budapest Treaty, on July 30, 2010, under accession number CNCM I-4348.

Preferably also, the glioma stem cell population according to the invention is the cell line deposited at the Collection Nationale de Culture de Micro-organismes (CNCM), Institut Pasteur, Paris, France, under the Budapest Treaty, on June 21, 2011, under accession number CNCM I-4496.

One of skill in the art knows many neural stem cell culture media comprising epidermal growth factor (EGF) and fibroblast growth factor 2 (FGF-2). Such a medium is notably described in reference (19). In particular, a neural stem cell culture media comprising epidermal growth factor (EGF) and fibroblast growth factor 2 (FGF-2) according to the invention comprises serum-free DMEM/F12 base medium supplemented with B27 supplement, heparin, and human recombinant epidermal growth factor (EGF) and fibroblast growth factor 2 (FGF-2), both at the final concentration of 20 ng/ml. As will be clear to one of skill in the art, it is preferred that the neural stem cell culture media of the invention do not comprise differentiation-inducing components, such as serum, in particular fetal bovine serum (FBS) or fetal calf serum (FCS).

Neurospheres are well-known to one of skill in the art and are notably described in reference (19). Neurospheres are usually defined as free-floating structures generated by neural stem cells or by cells presenting neural stem cells characteristics *in vitro.* A "neurosphere" according to the invention notably presents as a non-adherent cluster of cells, preferably spherical in shape. "Neurosphere populations" according to the invention relate to the neurospheres which are obtained after culturing the cells dissociated from the glioma tumor sample.

In a preferred embodiment of the method for obtaining a glioma stem cell population according to the invention, the neurosphere populations cultured according to the invention are passaged at least once, more preferably from at least once to at least 30 times, or at least 2, 3, 4, 5, 6, 7, 8, 9, 10, 15, 20, 25, 30, 35, 40, 50, 60, or 70 times. Passaging cells is a technique well known to one of skill in the art and may notably be defined as taking one or more cells from a cell culture and transferring the one or more cells into fresh cell culture medium, *i.e.* a cell culture medium which has not been used for cultivating cells. The culture duration between two passages can be easily defined by one of skill in the art depending on the type of cells to be cultured; the duration should preferably of a length sufficient to allow the cultured cells to divide, while not being so long as the cell culture medium to become unable to sustain optimal growth or mutliplication of the cultured cells, or the cultured cells to become senescent, for example. Preferably, the culture duration between two consecutive passages is of at least 5 days more preferably at least 6, 7, 8, 9, 10, 11, 12, 13, 14, or 15 days and most preferably of about 11 days.

In another preferred embodiment, the method for obtaining a glioma stem cell population according to the invention comprises:
- further determining that the tumor sample or the cells dissociated from the tumor sample display heterogeneous telomere lengths and/or an intracellular colocalization of telomeric DNA and promyelocytic leukemia (PML) nuclear bodies; and/or
- further selecting a neurosphere population displaying heterogeneous telomere lengths and/or an intracellular colocalization of telomeric DNA and promyelocytic leukemia (PML) nuclear bodies.

In another preferred embodiment of the method for obtaining a glioma stem cell population according to the invention, the individual presents a relapse of a glioblastoma multiforme, in particular at least a second or third relapse of a glioblastoma multiforme.

The anti-glioblastoma treatments to be screened according to the invention may be of any type. It is however preferred that they consist in candidate anti-glioblastoma drugs and/or radiations. The efficacy of anti-glioblastoma or anti-ALT treatments can be tested by treating the glioma stem cell population according of the invention *in vitro* and then assaying for changes in properties of the glioma stem cell population, such as tumor formation, tumor growth, tumor cell proliferation, tumor cell survival, or tumor cell cycle status. Assaying the changes may be performed *in vitro* or by transplanting the treated glioma stem cell population in a non-human animal, which is then monitored for tumor growth, cancer cell apoptosis, animal survival etc. A similar monitoring of the efficacy of the treatment can be performed when the candidate treatment is administered to an animal harboring glioblastoma tumors according to the invention. Besides, where the anti-glioblastoma or anti-ALT treatments consist of a compound, the compound can be attached to a solid surface as a microarray.

The invention will be further illustrated by the following non-limiting figures and Example.

### Description of the figures

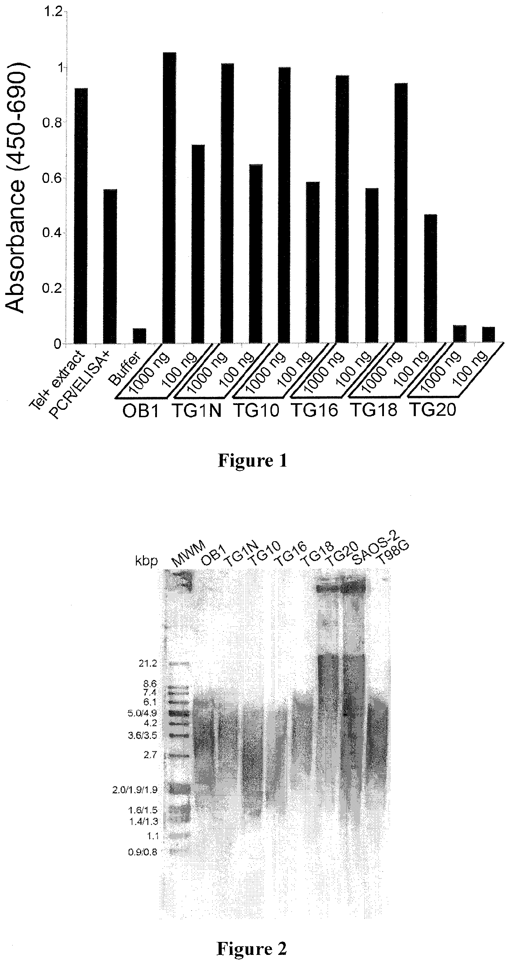

Figures 1 and 2: Determination of TG20 as a cell culture of the ALT type
Figure 1 represents telomerase activity as determined by PCR/ELISA (see Materials and Methods) in neurosphere cultures 7 days after seeding.
Figure 2 represents chemoluminescent detection of telomere restriction fragments (TRFs) of the designated cell lines by Southern blot using a dioxigenin labelled telomeric probe. MWM: Molecular Weight Marker.
Figures 3 and 4: *In vitro* expression of stem/differentiated cell markers in human glioma-derived ALT cultures

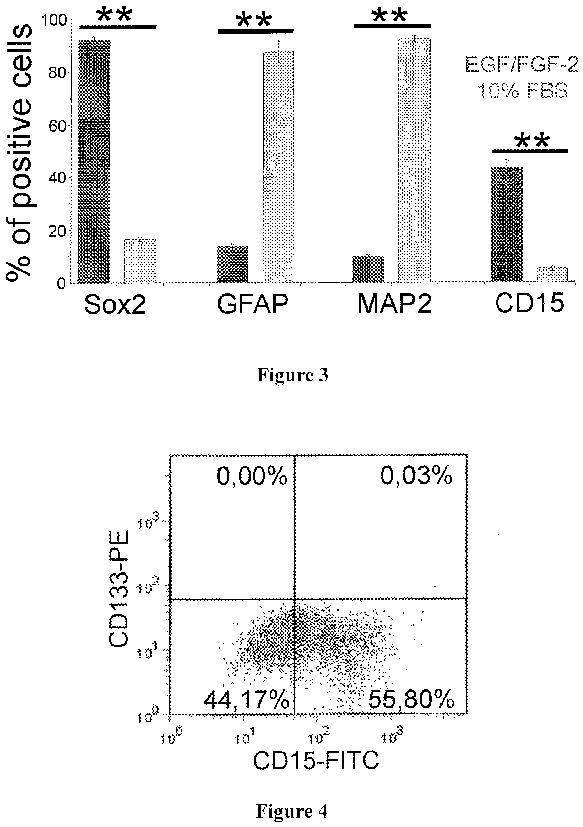

Figure 3 represents the quantification of cells expressing different stem cell-differentiation markers for GSCs cultured with EGF/FGF-2 (left column) or 10% fetal bovine serum (FBS). Error bars represent SEM (performed in triplicate, **p<0.005, as determined using Student's t test).
Figure 4 represents the flow cytometry analysis of neural stem cell surface marker expression on TG20 neurosphere cultures in proliferating conditions, using anti-CD 15 antibodies marked with fluoresceine (FITC) and anti-CD133 antibodies marked with Phycoerythrin (PE).

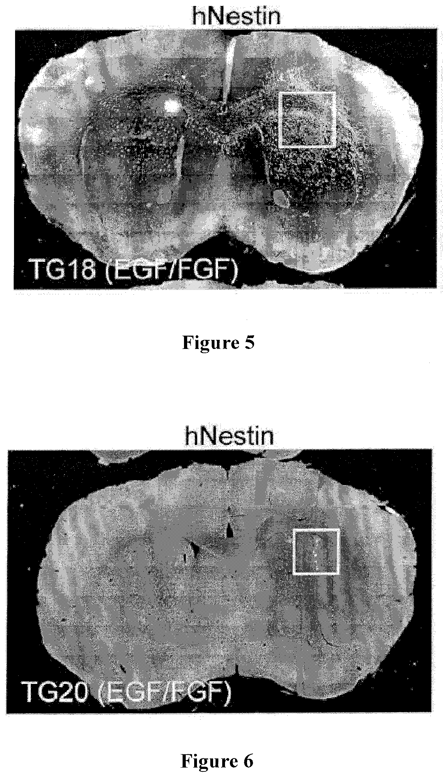

Figures 5, 6, and 7: *In vivo* cancer stem cell properties of human glioma-derived ALT cultures 100,000 cells from the specified adherent cultures were injected stereotaxically in the striatum of NOD-SCID-ILR2γ (NOG) mice. Ten weeks later, animals were sacrificed and the presence of grafted cells visualized by staining with the specified antibodies.
Figure 5 represents a photograph of a brain slice showing the administration site (white box) of TG18 cells cultured in stem cell conditions, human Nestin staining (n=3).
Figure 6 represents a photograph of a brain slice showing the administration site (white box) of TG20 cells cultured in stem cell conditions, human Nestin staining (n=4).

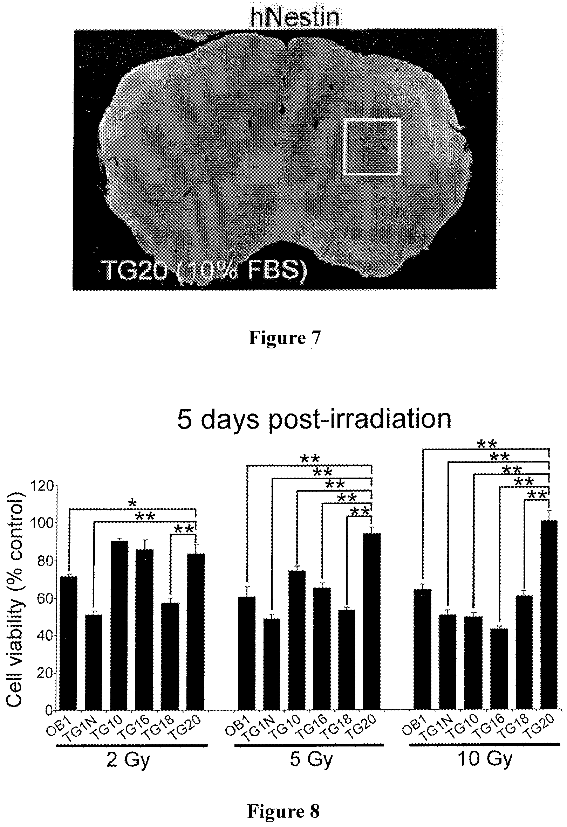

Figure 7 represents a photograph of a brain slice showing the administration site (white box) of TG20 cells cultured in medium containing 10% FBS for seven days before injection, human Nestin staining (n=3).

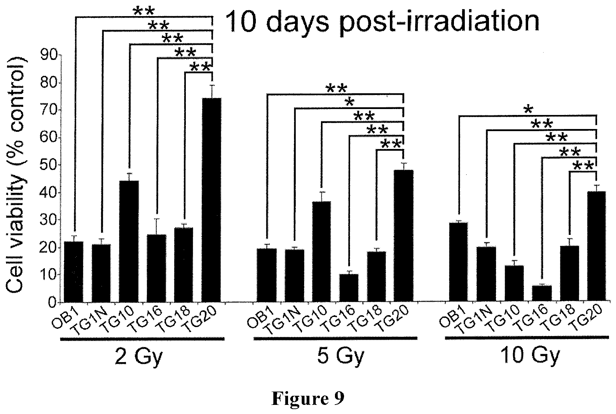

Figures 8 and 9: ALT GSC cells are less sensible to irradiation than telomerase+ counterparts Cells of the indicated cultures (4,000 cells/well) were seeded in laminin-coated 96-well. Five (Figure 9) and ten (Figure 10) days after irradiation, cell viability was determined by WST-1 assay. Each experimental condition was determined in tetraplicate. Percentages are the average ± SEM of 2 independent experiments. (*p<0.05, **p<0.005, as determined using Student's t test).

### EXAMPLE

### MATERIALS AND METHODS

### Cell cultures and treatment

Tumour samples were obtained from surgical resections of patients at the Sainte Anne hospital (Paris, France). Patients' informed consent was collected after approval of the Institutional Review Board. All tumors used in this study were diagnosed high-grade glioma (glioblastoma or oligoastrocytoma III), according to the WHO classification or Malignant Glio-Neuronal Tumor (MGNT) (19) according to the Sainte-Anne Hospital classification (Table I).

Glioma stem-like cells were obtained as previously described (19). Briefly, tumour samples were dissociated to form a single cell suspension, and plated in serum-free DMEM/F12 supplemented with B27 (Invitrogen), heparin (Stem Cell) and human recombinant epidermal growth factor (EGF) and fibroblast growth factor 2 (FGF-2) (Sigma), both at the final concentration of 20 ng/ml. Neurosphere cultures were then passaged every 11 days by mechanical dissociation to a concentration of 50,000 cells/mL in fresh medium in non-coated T25 or T75 flasks, for 70 consecutive passages. Cell counting was performed by Trypan Blue exclusion.

In some experiments, as specified in the figure legends, cells were grown in adherence in laminin (Sigma)-coated flasks using the same medium used for neurosphere cultures, as previously described (28).

For differentiation experiments, cells were cultured in Poly-L-Ornithine (Sigma) coated flasks in DMEM/F12 supplemented with 10% Fetal Bovine Serum (FBS) (Gibco).

Irradiations were performed using a ¹³⁷Cs source (IBL637, CIS BIO International) at a dose rate of 0.94 Gy/min in laminin-coated plates, 24 hrs after plating.

### Telomerase activity assay

Telomerase activity of neurosphere cultures in exponential growth (7 days after being passaged) was determined using the TRAPeze ELISA Telomerase Detection Kit (Chemicon), following the manufacturer's instructions. Protein concentrations were determined by the Bradford Method (BioRad).

### Telomere restriction fragments (TRF) Southern blot analysis

DNA was purified using DNA Isolation Kit for Cells and Tissues (Roche). Telomere restriction fragments were generated from 2 µg of total DNA by restriction with *Hinf*I and *Rsa*I (Roche). Separation on 0.8% agarose gels and visualization after hybridization with a telomere-specific, digoxigenin-labeled, probe was performed with the TeloTAGGG Telomere Length Assay Kit (Roche), following the manufacturer's instructions.

### PML/Telomeres colocalization and fluorescent in situ hybridization (FISH)

Dissociated neurospheres were collected by cytospin onto polylysine-treated glass slides. After 10 minutes of fixation in paraformaldehyde 4%, cells were permeabilized with PBS containing 0.1% Triton X-100 and 0.15% BSA for 5 minutes. Blocking was performed for 1 hour in PBS/7.5% BSA/7.5% FBS/ 0.1 Triton X-100. Cells were incubated overnight at 4°C with anti PML antibody (1/100, Santa Cruz), then incubated with FITC conjugated secondary antibody at room temperature for 1 hour. Hybridization with a telomeric Cyanine-3-conjugated (C3AT2)3 peptide nucleic acid (PNA) probe (Applied Biosystems) complementary to the G-rich telomeric strand was done using standard PNA-FISH procedures (46). Preparations were counterstained with DAPI and image acquisition done by confocal microscopy (DM 2500, Leica). Telomere-fluorescent *in situ* hybridization (Telo-FISH) was perfomed on metaphase spreads as previously described (47). Tumour biopsies were cryosectioned at 10 µm thickness and telomere/PML colocalization analysis performed as previously described (16), with minor modifications.

### FACS analysis.

Neurosphere cultures were mechanically disaggregated and stained with anti-CD133-PE (Miltenyi) and anti CD15-FITC (BD Biosciences) antibodies in a ratio of 5 µL of each antibody per 106 cells in a total volume of 100 µL for 30 minutes at 4°C. Isotype controls coupled to the same fluorophores as control antibodies were used in the same way. Data acquisition was performed on FACScalibur (BD Biosciences) and analysed using FlowJo software (Tree Star Inc.). For clonogenic analysis, cells were sorted into 96-well plates (1 cell/well) and analyzed one month later.

### Immunofluorescence analysis.

Cells were grown in Lab-Tek II Chambered Coverglass (Nunc), fixed with PFA 4% for 10 minutes at room temperature and permeabilized in Triton X-100 (Sigma) 0.1%. Primary antibodies were applied overnight at 4°C at the following dilutions: Sox2 (1:500, Chemicon), GFAP (1:400, Millipore) 04 (1:200, Millipore), MAP2 (1:100, Chemicon) and CD15 (1:50, BD Pharmingene). Secondary antibodies conjugated to FITC or Rhodamine were applied for 1 hour at room temperature (1:400, Molecular Probes). Preparations were counterstained with DAPI and image acquisition done by confocal microscopy (DM 2500, Leica).

### Xenotransplantation.

Glioma Stem cells (GSCs) were injected stereotaxically into 3-4 month-old NOD-SCID-IL2Rγ (NOG) mouse striatum, following administration of general anesthesia. 3 months later, animals were sacrificed and immunofluoresence analysis performed on brain slices using an anti-human Nestin antibody (1:400, R&D Systems) as previously described (28). All animal-related procedures were performed in accordance with the National Institute of Health Guide for the care and use of laboratory animals.

### RESULTS

### TG20 cells derived from a high grade glioma have an ALT phenotype

Although nearly 30% of Glioblastoma multiforme (GBM) have been reported to have an alternative lengthening of telomeres (ALT) phenotype, no glioma cell line activating the ALT pathway has been reported to date.

The inventors have thus measured telomerase activity using theTRAPeze ELISA Telomerase Detection Kit in glioma cell cultures grown under neural stem cell culture conditions that were isolated from 6 adult human glioma biopsies of unknown telomerase status (Table 1 and (19)).

Five of these glioma stem-like cultures (referred to as telomerase+ GSCs) displayed significant levels of telomerase activity, whereas this activity was undetectable in one, TG20 cells, isolated from a GBM at second relapse in a 38 year-old patient **(****Fig. 1****).**

The molecular basis of ALT is not yet known in detail, but involves telomere-telomere recombination (20-21), which leads to a unique pattern of telomere length heterogeneity.

Telomeric profile of TG20 cells was thus visualized by TRF Southern blot analysis. As shown in **Fig. 2****,** TG20 telomere length distribution resembles that of SAOS-2 cells, a well-known ALT osteosarcoma cell line (22), with long and heterogeneous telomere lengths (2 to 50 Kbs), whereas the telomerase+ GSCs showed the typical short and homogeneous telomeres of tumoral cells expressing telomerase, as the glioma cell line T98G used as control.

Heterogeneous telomere lengths of TG20 cells was further confirmed by Telo-FISH on metaphase chromosomes, showing strong heterogeneity in telomeric signals at chromatid ends, compared to telomerase+ GSCs. Moreover, TG20 cells showed abundant extrachromosomic telomeric DNA, another characteristic of ALT cells (23-24).

ALT cells are known to exhibit ALT-associated promyelocytic leukemia (PML) nuclear bodies (APBs), containing (TTAGGG)n DNA and telomere-specific binding proteins. APBs are a subset of PML bodies, present only in ALT cells and not found in telomerase positive cells (25). Colocalization of telomeric DNA and PML bodies, determined by confocal microscopy, confirmed the ALT phenotype of TG20 cells, whereas no colocalization was found in any of the telomerase+ glioma cultures.

Samples of the TG20 cells have been deposited at the Collection Nationale de Culture de Micro-organismes (CNCM), Institut Pasteur, Paris, France, under the Budapest Treaty, on July 30, 2010, under accession number CNCM I-4348, and on June 21, 2011, under accession number CNCM I-4496.

The inventors further performed an *in situ* analysis of the tumour sample from which TG20 were isolated to determine whether the original tumour presented also the ALT phenotype. Results showed the colocalization between telomeric DNA and PML bodies in tumour cells, indicating the ALT status of this GBM, and therefore that the ALT phenotype of TG20 cells is not a culture artefact but mirrors the ALT status of the original tumor.

Taken together, these results demonstrate that the TG20 cell population is the first culture model described to date of human ALT glioma.

### TG20 cells have the properties of cancer stem cells

The inventors also examined the cancer stem cell potential of TG20 cells.

The generally accepted criteria for GSCs are the ability to self-renew in defined stem cell culture conditions, the expression of normal neural stem cell markers, ability to generate differentiated progeny, and finally the capacity to initiate brain tumours in immunodeficient mice (4-5, 26-28).

TG20 cells have been grown for more than 70 passages as neurospheres, *ie.* in medium containing EGF and FGF2 as source of growth factors, which allows the proliferation of stem and progenitor cells (29). They showed a constant rate of proliferation with a population doubling time of 9.07±0.83 days (calculated from 16 passages), being on the range of the other telomerase+ GSC cultures involved in this study (ranging from 3.78 to 11.96 days).

Finally, clonogenicity assays - performed by seeding one cell per well in 96-well plates - evidenced that TG20 neurospheres contained 10% long-term colony-initiating cells.

Accordingly, these data demonstrate that TG20 cells have self-renewing capacity and thus that the ALT mechanism is able to confer, like telomerase activity, long-term proliferation capacity to glioma cells cultured in stem cell conditions.

Immunofluorescence staining of TG20 cells showed strong nuclear expression of Sox2, a transcription factor present in neural stem and progenitor cells during development and throughout adulthood and whose expression has been reported in GSCs (30).

By contrast, flow cytometry analysis did not allow the detection of CD133 expression by TG20 cells **(****Fig. 4****).** CD133 has been proposed as a marker of GSCs (5, 8), but several examples of GSCs lacking CD133 expression have been already reported (31-32).

Recently, Son *et al.* (33) have proposed CD15 as a more reliable GSC marker. The inventors found that approximately 40-60 % of TG20 cells expressed CD15 by flow cytometry and immunofluorescence assay **(****Fig. 3 and 4****).**

Altogether the data obtained by the inventors show that TG20 cells expressed neural stem cells markers such as Sox2 and CD 15.

Withdrawal of FGF2 and EGF and/or addition of 10% fetal bovine serum in culture medium induce the differentiation of GSCs (26, 34-37). As shown in **Fig. 3****,** in these conditions, TG20 cells lost Sox2 and CD15 expression. Conversely, most cells acquired the expression of both GFAP (87.58±4.1%) and MAP2 (92.45±1.19%), astrocyte and neuron markers, respectively, that were not detected in TG20 cells cultured in stem cell medium. No 04 expression, a marker of oligodendrocytes, was found in both conditions. TG20 cells are thus able to differentiate in cells expressing both astrocyte and neuron markers, loosing at the same time stem cell markers, an important feature of GSCs (28, 35, 38).

Finally, to test the capacity of TG20 cells to form tumors, intracranial transplantation was carried out into immuno-compromised NOD-SCID-IL2Rg (NOG) mice (Jackson Laboratories). The inventors injected 100,000 cells from TG18 (Telomerase+) and TG20 (ALT) cultures expanded in stem cell conditions.

*In vitro,* both cultures presented similar population doubling times (11.75±0.54 days for TG18 versus 9.07±0.83 days for TG20). Ten weeks after injection, mice were sacrificed, revealing large numbers of engrafted human Nestin immunoreactive cells that had infiltrated the host brain in the case of TG18 cells **(****Fig. 5****),** whereas animals injected with TG20 cells presented infiltrations of a much lesser extension and cells remained mainly near the injection site **(****Fig. 6****).**

As shown above, when cultured in the presence of FBS, TG20 cells lost the expression of stem cell markers and acquired the protein expression profile found on differentiated cells **(****Fig. 3****).**

It has been shown that GSCs differentiated by culturing in the presence of FBS lose their capacity to form intracranial tumors (26). Accordingly, the inventors tested if that was the case of TG20 cells. As can be clearly seen in **Fig. 7****,** no human Nestin positive cells were found 2 months after injecting 100,000 TG20 cells grown for 7 days in the presence of FBS, which clearly confirms that in this situation they lose their tumorigenic potential.

Taken together, the above results demonstrate that TG20 cells displayed the potential of GSCs according to the current paradigm.

In addition, the ability to generate and sustain tumors upon sequential transplantation has been reported to provide further evidence of a stem-like profile in human glioma cells (4,51).

Accordingly, the inventors carried out serial intracranial transplantations into severely immune deficient NOD-SCID-IL2Rγ (NOG) mice, using TG20 cells stably expressing the green fluorescent protein (TG20-GFP cells). Briefly, 70,000 cultured TG20-GFP cells were first inoculated into the brain of a first recipient NOG mouse to establish a tumor. After 3 month growth, 100,000 living TG20-GFP cells were sorted from the tumor and re-injected into a second recipient NOG mouse, which developed a tumor. Likewise, after 2 month growth, 100,000 living TG20-GFP cells were sorted form the tumor of the second recipient mouse and re-injected in a third recipient mouse, which also developed a tumor. Eventually, a tumor could be obtained in a fourth recipient mouse upon re-injection of 100,000 living TG20-GFP cells sorted from the tumor of the third recipient mouse, thereby confirming the GSC nature of TG20 cells.

In parallel, the inventors further confirmed that the capacity of TG20 cells to generate GFP-positive neurospheres *in vitro* was maintained throughout the performed transplantations.

Besides, it has been proposed that, with serial passages, changes in the phenotype of the sequential transplanted cells can occur (52). Indeed, while it has been reported that some tumor neural stem cells were able to maintain the same characteristics and karyotypic features of the first xenografted cells (4), differences in aggressiveness, tumor growth rate and invasiveness were described in other models (52), which could be due to an environmental influence or selection of particular cells upon transplantation.

Accordingly, the inventors have tested whether the TG20-GFP cells retained an ALT phenotype after *in vitro* transplantation. Applying the well-known Telomere Repeat Amplification Protocol (TRAP) assay, initially described by Kim et al. (1994) Science 266:2011-2015, the inventors have shown that no detectable telomerase activity could be evidenced in TG20-GFP cells recovered after the 3 successive transplantations.

### ALT glioma GSCs TG20 cells are more radiation resistant than their telomerase + counterparts

GSCs have been proposed to be responsible of repopulation of gliomas after chemo or radiotherapy (8, 39-40).

The inventors therefore compared the effect of gamma radiation on TG20 cells and on the 5 telomerase+ GSC cultures used in this work in order to determine whether the ALT mechanism could interfere with radiation response.

Cells cultured in adherent conditions as previously described (28) were irradiated with increasing doses of gamma rays from 2 to 10 Gy, 24 hrs after seeding (4,000 cells/well) on laminin-coated wells, and cell viability was measured 5 and 10 days after irradiation using the WST1 assay.

Results showed that TG20 cells were significantly more radio-resistant in comparison with the 5 telomerase+ GSCs. The effect could be seen starting from 5 Gy at 5 days post-irradiation and at all doses considered (from 2 to 10 Gy) 10 days post-irradiation **(****Fig. 8** **and** **9****).**

### CONCLUSION

In the present work, the inventors report the identification and characterization of the first cancer stem cell culture maintaining their telomeres by an alternative mechanism to telomerase.

Moreover, TG20 cells fulfill the widely accepted criteria for glioma stem cells: 1) capacity to proliferate *in vitro* in stem cell culture conditions, 2) expression of normal neural stem cell markers, 3) differentiation capacity and 4) initiation of intracranial tumours when injected in immunodeficient mice.

Therefore, these data show that the capacity to sustain long-term proliferation of cancer stem cells does not necessarily require telomerase activity, contrary to normal stem cells, but may also involve the ALT pathway.

The original tumour from which TG20 cells were obtained had an ALT phenotype, as nearly 30% of glioblastomas (15-16). Interestingly, this was a third resection of an original tumour that had been diagnosed 2 years before surgery (Table 1).

Furthermore, the inventors have shown that TG20 cells induced slowly progressing tumours in animals respect to telomerase+ counterparts. These data are thus consistent with the proposal of the ALT mechanism being an indicator of better prognosis of gliomas (16).

The obtained results thus demonstrates that ALT gliomas may also contain a subpopulation of GSC as already shown for telomerase+ gliomas (18, 41), which is important for understanding the development of this type of tumours.

Furthermore, TG20 cells are the first cultured cell model for ALT glioma, since no ALT glioma-derived cell line had been described before. Their capacity to initiate tumours in mice, establishing the first animal model of ALT glioma could be useful to develop specific therapeutic approaches.

A growing body of evidence suggests that cancer stem cells are major therapeutic targets (39). GSCs have been reported to be resistant to radiation because of preferential activation of DNA repair pathways compared to differentiated cancer cells, a process involving ATM, Rad17, Chk1 and Chk2 (8). The possible changes in cellular radiation response due to the activation of ALT pathway have been poorly investigated.

It is shown here that ALT GSC could be distinguished from telomerase+ GSCs by increased resistance to radiation, indicating that ALT mechanisms may directly affect therapeutic efficiency.

It may appear somewhat paradoxical that ALT phenotype of glioma tends to be associated both with a better prognosis as discussed above (16) and that ALT GSCs exhibit a greater resistance to treatment than telomerase + GSCs. However, aggressiveness and resistance to treatment are not necessarily linked. ALT mechanisms are presumably less efficient than telomerase to maintain telomeres, which thus could reduce the aggressiveness of tumours, whereas ALT could confer GSC a better resistance, consistently with TG20 cells being isolated from a third resection.

Relative radiation resistance of TG20 suggests that radiation-induced double-strand breaks (DSB) are processed differently in ALT cells compared to telomerase+ cells, which has been poorly investigated to date. ALT mechanism is dependent on homologous recombination at telomeres (for review see (21)). It is still unclear whether increased recombination activity occurs only at telomeres, since some minisatellite instability was found in ALT cells (42). However, against this hypothesis, no increase in sister chromatid exchange was observed elsewhere in the genome of ALT cells (43-44) and no increased activity has been reported using a recombination reporter assay (43). It has been recently shown that ALT cells present a spontaneous occurrence of telomeric DNA damage response (DDR) in the absence of chromosome fusions, differing in that from non-ALT cells (45). Cesare and Reddel (21) have proposed that ALT cells repress covalent ligation of telomeres eliciting DDR.

Further cytogenetic studies should investigate whether such mechanisms could interfere with DDR elicited by radiation-induced DSB in TG20 cells, repressing the generation of lethal chromosome aberrations.

TG20, as a new model of ALT cancer stem cells, will be useful for the improvement of the knowledge on the ALT mechanism and may thus help in the development of new therapeutic approaches in the treatment of ALT gliomas.

**Table I. Characteristics of patient tumors that generated neurosphere cultures used**

| **Patient Information** | | | | | |
|---|---|---|---|---|---|
| **GSC culture** | **Sex** | **Age (yr)** | **Diagnosis according to Saint-Anne Hospital classification (49)** | **Diagnosis according to WHO classification (48)** | **Treatment prior surgery** |
| OBI | F | 76 | MGNT | Oligoastrocytoma grade III | None |
| TG1N | M | 68 | MGNT | GBM | None |
| TG10 | M | 25 | MGNT | Giant cell GBM | None |
| TG16 | M | 67 | MGNT | Giant cell GBM | None |
| TG18 | F | 55 | MGNT | GBM | None |
| TG20 | M | 38 | MGNT relapse | GBM relapse | STUPP. 3 years prior relapse |

### REFERENCES

1. Furnari FB, et al. (2007) Malignant astrocytic glioma: genetics, biology, and paths to treatment. Genes Dev 21(21):2683-2710.
2. Laperriere N, Zuraw L, & Cairncross G (2002) Radiotherapy for newly diagnosed malignant glioma in adults: a systematic review. Radiother Oncol 64(3):259-273.
3. Zhu Y & Parada LF (2002) The molecular and genetic basis of neurological tumours. Nat Rev Cancer 2(8):616-626.
4. Galli R, et al. (2004) Isolation and characterization of tumorigenic, stem-like neural precursors from human glioblastoma. Cancer Res 64(19):7011-7021.
5. Singh SK, et al. (2004) Identification of human brain tumour initiating cells. Nature 432(7015):396-401.
6. Singh SK, et al. (2003) Identification of a cancer stem cell in human brain tumors. Cancer Res 63(18):5821-5828.
7. Rich JN (2007) Cancer stem cells in radiation resistance. Cancer Res 67(19):8980-8984.
8. Bao S, et al. (2006) Glioma stem cells promote radioresistance by preferential activation of the DNA damage response. Nature 444(7120):756-760.
9. de Lange T (2002) Protection of mammalian telomeres. Oncogene 21(4):532-540.
10. Greider CW & Blackburn EH (1985) Identification of a specific telomere terminal transferase activity in Tetrahymena extracts. Cell 43(2 Pt 1):405-413.
11. Klapper W, Shin T, & Mattson MP (2001) Differential regulation of telomerase activity and TERT expression during brain development in mice. J Neurosci Res 64(3):252-260.
12. Kruk PA, Balajee AS, Rao KS, & Bohr VA (1996) Telomere reduction and telomerase inactivation during neuronal cell differentiation. Biochem Biophys Res Commun 224(2):487-492.
13. Bryan TM, Englezou A, Gupta J, Bacchetti S, & Reddel RR (1995) Telomere elongation in immortal human cells without detectable telomerase activity. EMBO J 14(17):4240-4248.
14. Henson JD, Neumann AA, Yeager TR, & Reddel RR (2002) Alternative lengthening of telomeres in mammalian cells. Oncogene 21(4):598-610.
15. Hakin-Smith V, et al. (2003) Alternative lengthening of telomeres and survival in patients with glioblastoma multiforme. Lancet 361(9360):836-838.
16. Henson JD, et al. (2005) A robust assay for alternative lengthening of telomeres in tumors shows the significance of alternative lengthening of telomeres in sarcomas and astrocytomas. Clin Cancer Res 11(1):217-225.
17. Chen YJ, et al. (2006) Association of mutant TP53 with alternative lengthening of telomeres and favorable prognosis in glioma. Cancer Res 66(13):6473-6476.
18. Marian CO, et al. (2010) The telomerase antagonist, imetelstat, efficiently targets glioblastoma tumor-initiating cells leading to decreased proliferation and tumor growth. Clin Cancer Res 16(1):154-163.
19. Patru C, et al. (2010) CD133, CD15/SSEA-1, CD34 or side populations do not resume tumor-initiating properties of long-term cultured cancer stem cells from human malignant glio-neuronal tumors. BMC Cancer 10:66.
20. Dunham MA, Neumann AA, Fasching CL, & Reddel RR (2000) Telomere maintenance by recombination in human cells. Nat Genet 26(4):447-450.
21. Cesare AJ & Reddel RR (2010) Alternative lengthening of telomeres: models, mechanisms and implications. Nat Rev Genet 11(5):319-330.
22. Bryan TM, Englezou A, Dalla-Pozza L, Dunham MA, & Reddel RR (1997) Evidence for an alternative mechanism for maintaining telomere length in human tumors and tumor-derived cell lines. Nat Med 3(11):1271-1274.
23. Cesare AJ & Griffith JD (2004) Telomeric DNA in ALT cells is characterized by free telomeric circles and heterogeneous t-loops. Mol Cell Biol 24(22):9948-9957.
24. Wang RC, Smogorzewska A, & de Lange T (2004) Homologous recombination generates T-loop-sized deletions at human telomeres. Cell 119(3):355-368.
25. Yeager TR, et al. (1999) Telomerase-negative immortalized human cells contain a novel type of promyelocytic leukemia (PML) body. Cancer Res 59(17):4175-4179.
26. Lee J, et al. (2006) Tumor stem cells derived from glioblastomas cultured in bFGF and EGF more closely mirror the phenotype and genotype of primary tumors than do serum-cultured cell lines. Cancer Cell 9(5):391-403.
27. Piccirillo SG, et al. (2006) Bone morphogenetic proteins inhibit the tumorigenic potential of human brain tumour-initiating cells. Nature 444(7120):761-765.
28. Pollard SM, et al. (2009) Glioma stem cell lines expanded in adherent culture have tumor-specific phenotypes and are suitable for chemical and genetic screens. Cell Stem Cell 4(6):568-580.
29. Reynolds BA & Weiss S (1992) Generation of neurons and astrocytes from isolated cells of the adult mammalian central nervous system. Science 255(5052):1707-1710.
30. Hemmati HD, et al. (2003) Cancerous stem cells can arise from pediatric brain tumors. Proc Natl Acad Sci U S A 100(25):15178-15183.
31. Beier D, et al. (2007) CD133(+) and CD133(-) glioblastoma-derived cancer stem cells show differential growth characteristics and molecular profiles. Cancer Res 67(9):4010-4015.
32. Chen R, et al. (2010) A hierarchy of self-renewing tumor-initiating cell types in glioblastoma. Cancer Cell 17(4):362-375*.*
33. Son MJ, Woolard K, Nam DH, Lee J, & Fine HA (2009) SSEA-1 is an enrichment marker for tumor-initiating cells in human glioblastoma. Cell Stem Cell 4(5):440-452.
34. Gage FH, Ray J, & Fisher LJ (1995) Isolation, characterization, and use of stem cells from the CNS. Annu Rev Neurosci 18:159-192.
35. Lee J, et al. (2008) Epigenetic-mediated dysfunction of the bone morphogenetic protein pathway inhibits differentiation of glioblastoma-initiating cells. Cancer Cell 13(1):69-80.
36. McKay R (1997) Stem cells in the central nervous system. Science 276(5309):66-71.
37. Nakano I & Kornblum HI (2006) Brain tumor stem cells. Pediatr Res 59(4 Pt 2):54R-58R.
38. Wurdak H, et al. (2010) An RNAi screen identifies TRRAP as a regulator of brain tumor-initiating cell differentiation. Cell Stem Cell 6(1):37-47.
39. Frank NY, Schatton T, & Frank MH (2010) The therapeutic promise of the cancer stem cell concept. J Clin Invest 120(1):41-50.
40. Zhou BB, et al. (2009) Tumour-initiating cells: challenges and opportunities for anticancer drug discovery. Nat Rev Drug Discov 8(10):806-823.
41. Shervington A, Lu C, Patel R, & Shervington L (2009) Telomerase downregulation in cancer brain stem cell. Mol Cell Biochem 331(1-2):153-159.
42. Jeyapalan JN, et al. (2005) Activation of the ALT pathway for telomere maintenance can affect other sequences in the human genome. Hum Mol Genet 14(13):1785-1794.
43. Bechter OE, Shay JW, & Wright WE (2004) The frequency of homologous recombination in human ALT cells. Cell Cycle 3(5):547-549.
44. Londono-Vallejo JA, Der-Sarkissian H, Cazes L, Bacchetti S, & Reddel RR (2004) Alternative lengthening of telomeres is characterized by high rates of telomeric exchange. Cancer Res 64(7):2324-2327.
45. Cesare AJ, et al. (2009) Spontaneous occurrence of telomeric DNA damage response in the absence of chromosome fusions. Nat Struct Mol Biol 16(12): 1244-1251.
46. Pennarun G, et al. (2008) Role of ATM in the telomere response to the G-quadruplex ligand 360A. Nucleic Acids Res 36(5):1741-1754.
47. Pennarun G, et al. (2010) ATR contributes to telomere maintenance in human cells. Nucleic Acids Res 38(9):2955-2963.
48. Varlet P, et al. (2004) New variants of malignant glioneuronal tumors: a clinicopathological study of 40 cases. Neurosurgery 55(6):1377-1391: discussion 1391-1372.
49. Louis DN, et al. (2007) The 2007 WHO classification of tumours of the central nervous system. Acta Neuropathol 114(2):97-109*.*
50. Pearson et al. (2008) Curr Top Microbiol Immunol. 324:25-51.
51. Shu et al. (2008) Stem Cells 26:1414-24.
52. Strojnik et al. (2006) Anticancer Res. 26:2887-900.
53. Shervington et al., Brain Research, 2007, 45-52.
54. Langford et al., The Lancet, 1995, 346, 1267-1268.

## Claims

1. A glioma stem cell (GSC) line, wherein the glioma stem cells of the line do not present a telomerase activity.

2. The glioma stem cell line according to claim 1, wherein the glioma stem cells of the line display heterogeneous telomere lengths and/or an intracellular colocalization of telomeric DNA and promyelocytic leukemia (PML) nuclear bodies.

3. The glioma stem cell line according to claim 1 or 2, wherein the line is liable to generate glioblastoma tumors upon administration to an animal.

4. The glioma stem cell line according to any of claims 1 to 3, wherein less than 1% of the glioma stem cells of the line express CD133.

5. The glioma stem cell line according to any of claims 1 to 4, wherein from 30% to 70% of the glioma stem cells of the line express CD 15.

6. The glioma stem cell line according to any of claims 1 to 5, wherein more than 90% of the glioma stem cells of the line express Sox2.

7. The glioma stem cell line according to any of claims 1 to 6, deposited at the Collection Nationale de Culture de Micro-organismes (CNCM), Institut Pasteur, Paris, France, under the Budapest Treaty, on July 30, 2010, under accession number CNCM I-4348, or on June 21, 2011, under accession number CNCM I-4496.

8. A method for obtaining a glioma stem cell line, comprising:
- culturing cells dissociated from a glioma tumor sample obtained from an individual, in a neural stem cell culture medium comprising epidermal growth factor (EGF) and fibroblast growth factor 2 (FGF-2) to obtain neurosphere lines;
- determining that the tumor sample or the cells dissociated from the tumor sample do not present a telomerase activity and/or selecting a neurosphere line which does not present a telomerase activity;
thereby obtaining a glioma stem cell line.

9. The method of claim 8, wherein the neurosphere lines are passaged at least once.

10. The method according to claim 8 or 9, comprising further determining that the tumor sample or the cells dissociated from the tumor sample display heterogeneous telomere lengths.

11. The method according to any one of claims 8 to 10, comprising further determining that the tumor sample or the cells dissociated from the tumor sample display an intracellular colocalization of telomeric DNA and promyelocytic leukemia (PML) nuclear bodies.

12. The method according to any one of claims 8 to 11, comprising further selecting a neurosphere line displaying heterogeneous telomere lengths.

13. The method according to any one of claims 8 to 12, comprising further selecting a neurosphere line displaying an intracellular colocalization of telomeric DNA and promyelocytic leukemia (PML) nuclear bodies.

14. The method according to any of claims 8 to 13, wherein the individual presents a relapse of a glioblastoma multiforme.

15. The glioma stem cell line according to any of claims 1 to 7, which is obtainable by the method according to any of claims 8 to 14.

16. The use of a glioma stem cell line according to any of claims 1 to 7 and 15, for the generation of glioblastoma tumors in a non-human animal.

17. The use of a glioma stem cell line as defined in any of claims 1 to 7 and 15, for screening anti-glioblastoma treatments.

## Patentansprüche

1. Linie von Gliom-Stammzellen (GSC), wobei die Gliom-Stammzellen der Linie keine Telomerase-Aktivität aufweisen.

2. Gliom-Stammzellenlinie nach Anspruch 1, wobei die Gliom-Stammzellen der Linie heterogene Telomerlängen und/oder eine intrazelluläre Kolokalisierung von Telomer-DNA und Promyelozytenleukämie (PML)-Kernkörpern aufweisen.

3. Gliom-Stammzellenlinie nach Anspruch 1 oder 2, wobei die Linie bei Verabreichung an ein Tier für die Erzeugung von Glioblastom-Tumoren verantwortlich ist.

4. Gliom-Stammzellenlinie nach einem der Ansprüche 1 bis 3, wobei weniger als 1 % der Gliom-Stammzellen der Linie CD133 exprimieren.

5. Gliom-Stammzellenlinie nach einem der Ansprüche 1 bis 4, wobei 30 % bis 70 % der Gliom-Stammzellen der Linie CD15 exprimieren.

6. Gliom-Stammzellenlinie nach einem der Ansprüche 1 bis 5, wobei mehr als 90 % der Gliom-Stammzellen der Linie Sox2 exprimieren.

7. Gliom-Stammzellenlinie nach einem der Ansprüche 1 bis 6, hinterlegt bei der Collection Nationale de Culture de Microorganismes (CNCM), Institut Pasteur, Paris, Frankreich, gemäß dem Budapester Abkommen am 30. Juli 2010 unter der Zugangsnummer CNCM I-4348 oder am 21. Juni 2011 unter der Zugangsnummer CNCM I-4496.

8. Verfahren zur Gewinnung einer Gliom-Stammzellenlinie, umfassend:
- Kultivieren von Zellen, die aus einer Gliom-Tumorprobe dissoziiert wurden, die aus einem Individuum gewonnen wurde, in einem Kulturmedium neuraler Stammzellen, das epidermalen Wachstumsfaktor (EGF) und Fibroblastenwachstumsfaktor 2 (FGF-2) umfasst, um Neurosphären-Linien zu gewinnen;
- Bestimmen, dass die Tumorprobe oder die aus der Tumorprobe dissoziierten Zellen keine Telomerase-Aktivität aufweist bzw. aufweisen, und/oder Auswählen einer Neurosphären-Linie, die keine Telomerase-Aktivität aufweist;
um dadurch eine Gliom-Stammzellenlinie zu gewinnen.

9. Verfahren nach Anspruch 8, wobei die Neurosphären-Linien mindestens einmal passagiert werden.

10. Verfahren nach Anspruch 8 oder 9, ferner umfassend ein Bestimmen, dass die Tumorprobe oder die aus der Tumorprobe dissoziierten Zellen heterogene Telomerlängen aufweist bzw. aufweisen.

11. Verfahren nach einem der Ansprüche 8 bis 10, ferner umfassend ein Bestimmen, dass die Tumorprobe oder die aus der Tumorprobe dissoziierten Zellen eine intrazelluläre Kolokalisierung von Telomer-DNA und Promyelozytenleukämie (PML)-Kernkörpern aufweist bzw. aufweisen.

12. Verfahren nach einem der Ansprüche 8 bis 11, ferner umfassend ein Auswählen einer Neurosphären-Linie, welche heterogene Telomerlängen aufweist.

13. Verfahren nach einem der Ansprüche 8 bis 12, ferner umfassend ein Auswählen einer Neurosphären-Linie, die eine intrazelluläre Kolokalisierung von Telomer-DNA und Promyelozytenleukämie (PML)-Kernkörpern aufweist.

14. Verfahren nach einem der Ansprüche 8 bis 13, wobei das Individuum ein Rezidiv eines *Glioblastoma multiforme* aufweist.

15. Gliom-Stammzellenlinie nach einem der Ansprüche 1 bis 7, die durch das Verfahren nach einem der Ansprüche 8 bis 14 gewonnen werden kann.

16. Verwendung einer Gliom-Stammzellenlinie nach einem der Ansprüche 1 bis 7 und 15 zur Erzeugung von Glioblastom-Tumoren in einem nichthumanen Tier.

17. Verwendung einer Gliom-Stammzellenlinie nach einem der Ansprüche 1 bis 7 und 15 zum Screening von Antiglioblastom-Behandlungen.

## Revendications

1. Lignée de cellules souches de gliome (GSC), dans laquelle les cellules souches de gliome de la lignée ne présentent pas d'activité de télomérase.

2. Lignée de cellules souches de gliome selon la revendication 1, dans laquelle les cellules souches de gliome de la lignée présentent des longueurs hétérogènes de télomère et/ou une colocalisation intracellulaire d'ADN télomérique et de corps nucléaires de leucémie promyélocytaire (PML).

3. Lignée de cellules souches de gliome selon la revendication 1 ou 2, dans laquelle la lignée est susceptible de générer des tumeurs de glioblastome après administration à un animal.

4. Lignée de cellules souches de gliome selon l'une quelconque des revendications 1 à 3, dans laquelle moins de 1 % des cellules souches de gliome de la lignée exprime CD133.

5. Lignée de cellules souches de gliome selon l'une quelconque des revendications 1 à 4, dans laquelle de 30 % à 70 % des cellules souches de gliome de la lignée expriment CD15.

6. Lignée de cellules souches de gliome selon l'une quelconque des revendications 1 à 5, dans laquelle plus de 90 % des cellules souches de gliome de la lignée expriment Sox2.

7. Lignée de cellules souches de gliome selon l'une quelconque des revendications 1 à 6, déposée à la Collection Nationale de Culture de Micro-organismes (CNCM), Institut Pasteur, Paris, France, en vertu du traité de Budapest, le 30 juillet 2010, sous le numéro d'accès CNCM I-4348, ou le 21 juin 2011, sous le numéro d'accès CNCM I-4496.

8. Procédé d'obtention d'une lignée de cellules souches de gliome, comprenant :
- la culture de cellules dissociées d'un échantillon de tumeur de gliome obtenu à partir d'un individu, dans un milieu de culture de cellules souches neurales comprenant le facteur de croissance épidermique (EGF) et le facteur 2 de croissance des fibroblastes (FGF-2) pour obtenir des lignées de neurosphères ;
- la détermination que l'échantillon de tumeur ou les cellules dissociées de l'échantillon de tumeur ne présentent pas une activité de télomérase et/ou la sélection d'une lignée de neurosphères qui ne présente pas une activité de télomérase ;
en obtenant ainsi une lignée de cellules souches de gliome.

9. Procédé selon la revendication 8, dans lequel les lignées de neurosphères sont repiquées au moins une fois.

10. Procédé selon la revendication 8 ou 9, comprenant en outre la détermination que l'échantillon de tumeur ou les cellules dissociées de l'échantillon de tumeur présentent des longueurs hétérogènes de télomère.

11. Procédé selon l'une quelconque des revendications 8 à 10, comprenant en outre la détermination que l'échantillon de tumeur ou les cellules dissociées de l'échantillon de tumeur présentent une colocalisation intracellulaire d'ADN télomérique et de corps nucléaires de leucémie promyélocytaire (PML).

12. Procédé selon l'une quelconque des revendications 8 à 11, comprenant en outre la sélection d'une lignée de neurosphères présentant des longueurs hétérogènes de télomère.

13. Procédé selon l'une quelconque des revendications 8 à 12, comprenant en outre la sélection d'une lignée de neurosphères présentant une colocalisation intracellulaire d'ADN télomérique et de corps nucléaires de leucémie promyélocytaire (PML).

14. Procédé selon l'une quelconque des revendications 8 à 13, dans lequel l'individu présente une récidive d'un glioblastome multiforme.

15. Lignée de cellules souches de gliome selon l'une quelconque des revendications 1 à 7, qui peut être obtenue par le procédé selon l'une quelconque des revendications 8 à 14.

16. Utilisation d'une lignée de cellules souches de gliome selon l'une quelconque des revendications 1 à 7 et 15, pour la génération de tumeurs de glioblastome chez un animal non humain.

17. Utilisation d'une lignée de cellules souches de gliome telle que définie dans l'une quelconque des revendications 1 à 7 et 15, pour le criblage de traitements anti-glioblastome.
